# EUROPEAN PATENT SPECIFICATION

(11) **EP 1 364 614 B1**
(45) Date of publication and mention of the grant of the patent: **08.07.2009**
(21) Application number: 03011025.8
(22) Date of filing: 19.05.2003
(51) Int. Cl.: A61B 5/0492, G01R 19/00

(54) **Voltage measuring device comprising fixing member, electrode and transmitter**
Volt-Messgerät mit Halterung, Elektrode und Signalüberträger
Appareil de mesure de tension avec élément de maintien, électrode et émetteur

(30) Priority: 20.05.2002 JP 2002145384
(43) Date of publication of application: 26.11.2003
(73) Proprietor: NTT DoCoMo, Inc., Tokyo 100-6150 (JP)
(72) Inventor: Manabe, Hiroyuki, Int Prop Dept, NTT DoCoMo, Inc., Chiyoda-ku, Tokyo (JP); Hiraiwa, Akira, Int Prop Dept, NTT DoCoMo, Inc., Chiyoda-ku, Tokyo (JP); Sugimura, Toshiaki, Int Prop Dept, NTT DoCoMo, Inc, Chiyoda-ku, Tokyo (JP)
(74) Representative: HOFFMANN EITLE

(56) References cited:
- EP-A- 0 435 416
- EP-A- 0 580 092
- CN-C- 1 213 567
- FR-A- 2 643 811
- US-A- 3 542 010
- US-A- 4 094 309
- US-A- 4 308 873
- US-A- 4 765 343
- US-A- 4 858 617
- US-A- 4 858 617
- US-A- 4 865 039

## Description

### BACKGROUND OF THE INVENTION

### 1. Field of the Invention

The present invention relates to a measuring device for measuring voltage.

### 2. Description of the Related Art

In document FR-A-2 643 811, there is described an annular electrode utilisable for the realisation of electrocardiograms. An armature is realised with an electrically conducting material having on one side a face to get an engagement, e.g., to a finger or an ear, and on the other side thereof means which permits the connection of an electrically conducting cable which may be connected to an electrocardiographic apparatus.

Also, in US-A-4, 765,343 there is described an apparatus for transferring electrical energy to and from living tissue comprising a glove of thin, flexible elastomeric material, which carries electrodes, electrically connected to a connector disposed at the cuff of the glove. The connector permits the electrodes to be electrically connected to a source of electrical energy or to a load, so that electrical energy may be applied to or drawn from living tissue.

In EP 0 435 416 A2 there is described an electrode glove for use in transcutaneous electrical nerve stimulation. The glove has an electrically-insulating skin and an optional moisture absorbent fabric liner being formed on a porcelain or metal form and coated entirely by an electrically-conductive, electro-chemically inert, flexible, elastomeric electrode. A connector containing holes accepting the pin on the distal end of the lead wire of a transcutaneous electrical nerve stimulation TENS unit is attached to the exterior surface of the electrode, during manufacture or by a clinician prior to use.

In US-A-4,865,039, there is described a dry electrode system for detection of biopotentials and dry electrode for making electrical and mechanical connection to a living body. The dry electrode system includes a dry electrode pad with a resilient conductive pad adhering to at least one adhesive pad or otherwise having opposed adhesive surfaces, one of which is adapted to engage the skin of a living body. The dry electrode pad makes electrical connection to an amplifying circuit which transmits a biopotential derived from the conductive pad to a monitor. The amplifying circuit includes a conductive input contact for making electrical contact to the conductive pad, a lead amplifier having an input coupled to the input contact, and a voltage-driven shield coupled to the output of the lead amplifier and surrounding portions of the input contact not in engagement with the dry electrode pad. In a preferred embodiment, conductive adhesive layers are applied to opposed sides of a conductive foam pad to construct the dry electrode pad.

In US-A-4,858,617, there is described a cardiac probe enabling use of personal computer for monitoring heart activity or the like. The probe unit has projecting electrodes for sensing minute voltage variations at spaced apart locations on a person's skin or other surface. Internal circuits generate serial form digital signals indicative of the voltage variations for transmission to a personal computer where an electrocardiogram or other data presentation may be displayed.

In US-A-3,542,010, there is described a surface contacting electrode assembly having an electrically conductive pile forming contact surface. The electrode assembly is formed of an electrically conductive, matted-loop pile carried on a surface of an elongated, flexible fabric strip of a length to encircle a body member for positioning of the pile in electrical contact-forming engagement with the skin surface. A multiplicity of hooks formed on an opposite surface of the fabric strip and releasably engageable with the loop file form fastening means when interengaged to facilitate securance of the assembly to a body member. A connector terminal secured to the fabric strip and in electrical contact with the pile facilitates connection of the electrode assembly with associated diagnostic apparatus.

In order to measure biological information such as electromyography (EMG) or brain waves, an electrode is used for measuring variation in voltage on the skin of a human body, which is a measurement object. Conventionally, a metallic plate electrode is used as the electrode. The plate electrode is applied with paste for reducing contact impedance between the electrode and the skin. The plate electrode is attached to the skin of the human body and is fixed with tape. Then, the plate electrode measures variation in voltage on the skin while being fixed with the tape. A single-use electrode called a disposable electrode is also used, which is designed to impart adhesiveness to an electrode itself or a surrounding member of the electrode so that the electrode contacts the skin without fixation by use of tape, and thereby measures the variation in voltage on the skin.

However, in a measuring device using the plate electrode fixed with tape, the attached electrode can become dislocated or an appropriate state of contact with the surface of the skin cannot be retained, when the skin (which is the measurement object) moves, because the electrode is fixed with tape. As a result, the measuring device cannot follow movements of the measurement object. In addition, the operation of attaching the electrode with tape is complicated.

Meanwhile, in the case of the disposable electrode, it is necessary to enhance the adhesiveness of the electrode or the surrounding member, or to increase the contact area of the electrode with the skin, so that the electrode can retain a state of contact, without coming away from the skin despite any movement of the skin, which is the measurement object. However, when the adhesiveness is enhanced, the disposable electrode may cause adverse effects on the measurement object, such as the skin. Further, when the contact area is increased, the disposable electrode cannot follow delicate movements, of the measurement object. In addition, the disposable electrode is not reusable.

### BRIEF SUMMARY OF THE INVENTION

An object of the present invention is to provide a measuring device, which is capable of following the movement of a measurement object, and appropriately measuring a necessary voltage.

According to the present invention this object is achieved by a measuring device having the features of claim 1.

The above-described measuring device comprises the fixing member, and the electrode is mounted on the opposite side of the fixing member to the contact surface with the fixation object. Therefore, the measuring device is fixed to the fixation object with the fixing member, and it is thereby possible to measure by pressing the electrode against the measurement object. Hence, the measuring device can follow the movement of the measurement object sufficiently. In addition, the fixing member has the nonconductive contact surface with the fixation object. Accordingly, the fixing member can electrically insulate between the fixation object and the electrode. Therefore, the electrode can measure only the necessary voltage on the measurement object appropriately without suffering from the adverse effect of a voltage generated on the fixation object, i.e. an unnecessary voltage. Moreover, the measuring device can transmit the measured voltage externally via the transmitter. Accordingly, the voltage measured by the measuring device can be used for many purposes by detecting a signal from the voltage, and the like.

### BRIEF DESCRIPTION OF THE SEVERAL VIEWS OF THE DRAWINGS

Fig. 1 is a perspective view of a measuring device helpful for understanding the present invention.
Fig. 2 is a view showing a structure of the measuring device shown in Fig. 1.
Fig. 3 is a view showing a method of measuring voltage by use of the measuring device shown in Fig. 1.
Fig. 4 is a perspective view of a measuring device helpful for understanding the present invention.
Figs. 5A, 5B and 5C are views showing an active electrode-type measuring device helpful for understanding the present invention.
Fig.6 is a view showing a method of measuring voltage by use of the measuring device shown in Figs. 5A, 5B and 5C.
Fig. 7 is a perspective view of an active electrode-type measuring device helpful for understanding the present invention.
Fig. 8 is a perspective view of an active electrode-type measuring device not forming an embodiment of the present invention.
Fig. 9 is a perspective view of an active electrode-type measuring device not forming an embodiment of the present invention.
Fig. 10 is a perspective view of an active electrode-type measuring device not forming an embodiment of the present invention.
Fig. 11 is a perspective view of a measuring device not forming an embodiment of the present invention.

### DETAILED DESCRIPTION OF THE INVENTION

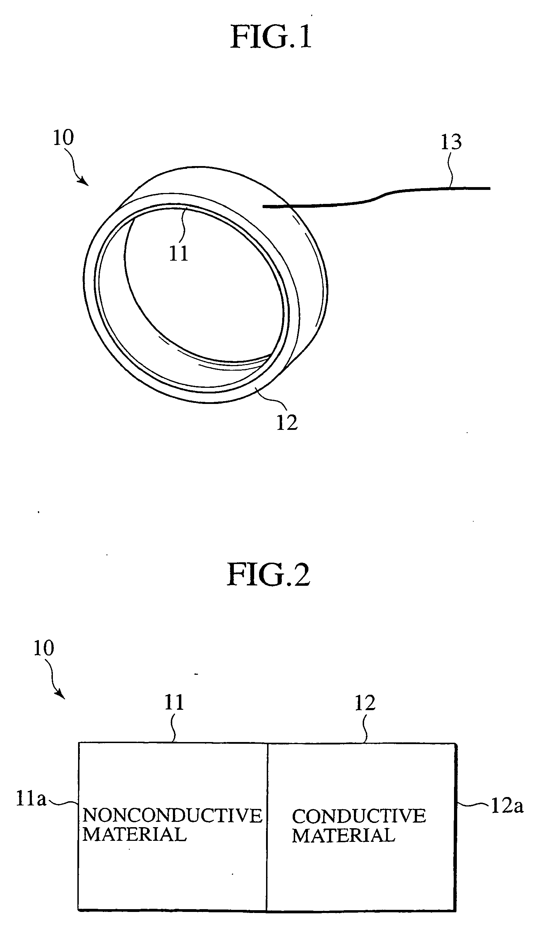

As shown in Fig. 1, a measuring device 10 comprises a fixing member 11, an electrode 12, and a lead wire 13. Moreover, as shown in Fig. 1 and Fig. 2, the measuring device 10 has a two-layer structure formed by the fixing member 11 and the electrode 12.

The fixing member 11 is fixed to a fixation object. The fixing member 11 is formed into an annular shape, which can be fitted to the fixation object. In other words, the fixing member 11 includes an annular portion configured to fit to the fixation object. The fixation object is an object to which the measuring device 10 is fixed. In the case of the measuring device 10, the fixation object is a finger of a human body. Accordingly, the fixing member 11 is formed into an annular shape to be a ring shape, which can be fitted to the finger. It is preferable that the inside diameter of the fixing member 11 is approximately equal to the outside diameter of a fixing portion of the fixation object so that the fixing member 11 can be stably fixed to the fixation object. However, the inside diameter of the fixing member 11 needs to be determined within a range such that the fixing member 11 is fitted to the fixation object and removed from the fixation object.

The fixing member 11 constitutes the inner side of the two-layer structure formed by the fixing member 11 and the electrode 12. Moreover, the fixing member 11 includes a contact surface, which contacts the fixation object when the fixing member is fixed to the fixation object. Specifically, the fixing member 11 includes the contact surface, which contacts the finger when the measuring device 10 is fitted to the finger. In the measuring device 10, the entire inner surface of the ring of the fixing member 11 serves as the contact surface. As shown in Fig. 2, the fixing member 11 is made of a nonconductive material. For this reason, a contact surface 11a to contact the finger, which is the fixation object, is made of the nonconductive material. Therefore, the measuring device 10 can electrically insulate between the finger and the electrode 12 by use of the fixing member 11. In this way, the fixing member 11 has the nonconductive contact surface 11a in contact with the fixation object. For the nonconductive material for forming the fixing member 11, it is possible to use insulation such as plastic, resin or rubber, for example. Further, for the fixing member 11, it is possible to use a solid type, a sheet type, or a thin-film type formed by coating the inner side surface of the ring of the electrode 12.

The fixing member 11 holds the electrode 12. To be more specific, the single electrode 12 is mounted on the opposite side of the fixing member 11 to the contact surface 11a, that is, on an outer side of the ring of the fixing member 11. Accordingly, it is preferable that the fixing member 11 has a width sufficient for mounting the single electrode 12 and preventing contact between the finger and the electrode 12. For example, the width of the fixing member 11 can be set equal to or wider than the width of the electrode 12. In the measuring device 10, the width of the fixing member 11 is equal to the width of the electrode 12.

The electrode 12 measures voltage on a measurement object. As shown in Fig. 1, the single electrode 12 is mounted on the opposite side of the fixing member 11 to the contact surface 11a with the finger, which is the fixation object. To be more specific, the electrode 12 is formed into an annular shape for covering the outer side of the ring of the fixing member 11, and is mounted on the opposite side to the contact surface 11a. The electrode 12 has the same width as that of the fixing member 11, and both ends of the electrode 12 and both ends of the fixing member 11 are equal to one another when the measuring device 10 is viewed from above. Viewing the measuring device 10 from above is equivalent to viewing the measuring device 10 from the direction where the ring of the measuring device 10 is invisible. Moreover, when the width of the electrode 12 is smaller than the width of the fixing member 11, the electrode 12 is mounted on the fixing member 11 so as to be located inside the width direction of the fixing member 11. In this way, it is possible to prevent the electrode 12 from contacting the fixation object when the measuring device 10 is fixed to the fixation object.

The electrode 12 forms the outer side of the two-layer structure formed by the fixing member 11 and the electrode 12. Moreover, the electrode 12 includes a measurement surface 12a, which contacts the measurement object and measures electric potential. In the measuring device 10, the entire surface of the electrode 12 of the opposite side to a contact surface with the fixing member 11 serves as the measurement surface 12a. The measurement object is an object on which voltage is measured by the measuring device 10. The measurement object includes the skin on a surface of part of a human body such as a head, a face, an arm, or a leg. If the measurement object is the skin on the surface of the head, it is possible to detect brain waves from the variation in the measured voltage. If the measurement object is the skin on the surface of the face such as the mouth, on the surface of the arm, or on the surface of the leg, it is possible to detect EMG from the variation in the measured voltage.

As shown in Fig. 2, the electrode 12 is made of a conductive material. For this reason, the measurement surface 12a to contact the skin, which is the measurement object, on the surface of the head or the face, is made of conductive material. Accordingly, voltage is induced to the electrode when the measurement surface 12a is pressed against the measurement object to make contact. In this way, the electrode 12 can measure the voltage. For the conductive material for forming the electrode 12, it is possible to use silver, platinum or silver chloride, for example. Meanwhile, for the electrode 12, it is possible to use a solid type, a sheet type, or a thin-film type formed by coating the surface of the outer side of the ring of the fixing member 11.

Therefore, if both the fixing member 11 and the electrode 12 are of the solid types, then it is possible to form the ring shape of the two-layer structure including the fixing member 11 and the electrode 12 by fitting the fixing member 11 into the inner side of the ring of the electrode 12. Further, if both the fixing member 11 and the electrode 12 are of the sheet types, then it is possible to form the ring shape of the two-layer structure including the fixing member 11 and the electrode 12 by attaching the fixing member 11 to the inner side of the ring of the electrode 12. Further, if the fixing member 11 is of the solid type, and the electrode 12 is of the sheet type or the thin-film type, then it is possible to form the ring shape of the two-layer structure including the fixing member 11 and the electrode 12 by attaching the electrode 12 of the sheet type to the outer surface of the ring of the fixing member 11, or by coating the outer surface of the ring of the fixing member 11 with the material for the electrode 12 to form the electrode 12 of the thin-film type. On the other hand, if the fixing member 11 is of the sheet type or the thin-film type, and the electrode 12 is of the solid type, then it is possible to form the ring shape of the two-layer structure including the fixing member 11 and the electrode 12 by attaching the fixing member 11 of the sheet type to the inner surface of the ring of the electrode 12 or by coating the inner surface of the ring of the electrode 12 with the material for the fixing member 11 to form the fixing member 11 of the thin-film type.

The lead wire 13 is a transmitter configured to transmit the voltage measured by the electrode 12 externally. One end of the lead wire 13 is connected to part of the measurement surface 12a of the electrode 12. The other end of the lead wire 13 is connected to an external device. The external device to which the lead wire 13 is connected is an amplifier, a computing unit, or an output device, for example. The amplifier amplifies the voltage measured by the electrode 12. The computing unit processes by use of a signal based on the voltage measured by the electrode 12. For example, the computing unit detects the signal from the measured voltage, and performs speech recognition processing for recognizing a voice or recognition processing for recognizing a motion or an action by use of the signal. The output device includes a display or a printer for outputting the measured voltage or the signal detected from the voltage as a measured value.

Moreover, the lead wire 13 transmits the voltage measured by the electrode 12 and delivers the voltage to the external device such as the amplifier, the computing unit, or the output device. The amplifier or the computing unit can detect biological information such as brain waves or EMG from the variation in the voltage of the measurement object, which is received from the measuring device 10.

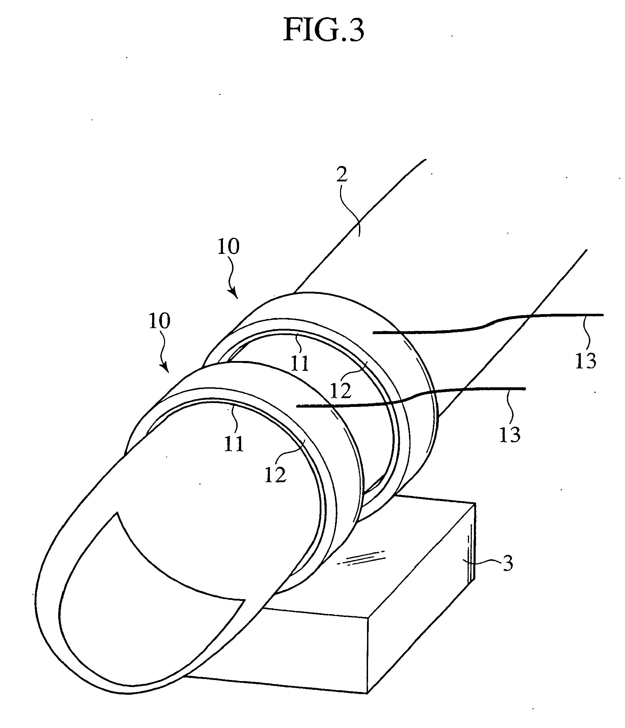

Next, a description will be made regarding a method of measuring voltage by use of the measuring device 10. As shown in Fig. 3, a subject of the voltage measurement puts the fixing members 11 of two measuring devices 10 on one finger 2. In this way, the fixing members 11 are fixed to the finger 2. Next, the subject presses the electrodes 12 against a region targeted for measurement, such as the skin 3 on the surface of the head, of the face, of the arm, or of the leg to make contact. In this way, voltage is induced between the two electrodes 12, and the electrodes 12 thereby measure the voltage. The lead wires 13 transmit the measured voltage and deliver the voltage to an external device such as the amplifier, the computing unit, the output device, or the like. Accordingly, the measured voltage is transmitted to the external device through the lead wires 13. The external device such as the amplifier or the computing unit detects variation of a difference in the voltage between the two electrodes 12 (this difference will be hereinafter referred to as "electric potential difference", and this variation will be hereinafter referred to as "electric potential variation"), and detects biological information such as brain waves or EMG from the electric potential variation. Then, the computing unit performs the speech recognition processing or the processing for recognizing amotion or an action by use of the biological information. Meanwhile, the output device outputs the measured voltage and the detected biological information as measured values.

Here, the number of the measuring devices 10 to be fixed to the fixation object (the finger 2 of the subject) is not limited. However, since the measuring device 10 includes the single electrode 12, as many of the measuring devices 10 as the number of measurement places are fixed to the fixation object, if the signals are detected by differential amplification or if the voltage is measured in a plurality of places. Moreover, the position on the fixation object where the measuring device 10 is fixed is not limited either. Although the two electrodes 12 are fitted to a single finger 2 in Fig. 3, it is also possible to fit the electrodes 12 separately to two fingers.

The measuring device 10 comprises the fixing member 11, and the electrode 12 is mounted on the opposite side of the fixing member 11 to the contact surface 11a with the finger, which is the fixation object. Therefore, the measuring device 10 is fixed to the finger 2 with the fixing member 11 only by fitting the fixing member 11 to the finger 2. Accordingly, the measuring device 10 can easily measure the voltage by merely pressing the electrode 12 against the region targeted for measurement such as the skin 3 on the surface of the head, on the surface of the face, on the surface of the arm, or on the surface of the leg. As a result, even if the muscle of the head, of the face, of the arm, or of the leg moves and the skin thereof also moves, the measuring device 10 can sufficiently follow the movement and flexibly deal with the movement.

Moreover, since the fixing member 11 is formed into an annular shape when fitted to the finger 2 (i.e. the fixing member 11 includes the annular portion configured to fit to the finger 2), the electrode 12 can be easily and stably fixed to the fixation object such as the finger 2 without using tape, by fitting the annular fixing member 11 to the finger 2. In addition to this, upon measurement, all that is required is to press the electrode 12 against the measurement object. Accordingly, the electrode 12 itself or a surrounding member of the electrode 12 does not need to be adhesive. As described above, the measuring device 10 does not need to be fixed to the fixation object and the measurement object with tape or an adhesive. Therefore, it is possible to suppress adverse effects on the fixation object and the measurement object, such as adverse effects on the finger 2 and the skin 3 on the surface of the head, on the surface of the face, on the surface of the arm, or on the surface of the leg.

Furthermore, since the measuring device 10 can fix the electrode 12 to the finger 2, it is easy to press the electrode 12 fixed to the finger 2 against the skin 3. Accordingly, the degree of contact of the electrode 12 with the measurement object can be easily adjusted by merely adjusting the degree of the above-described pressure. As a result, the measuring device 10 can deal with the movement of muscles more flexibly.

Therefore, the measuring device 10 can be utilized as a novel interface for use in the case of detecting biological information such as brain waves or EMG from the electric potential variation of the skin 3 on the surface of the head, on the surface of the face, on the surface of the arm, or on the surface of the leg, and analyzing and utilizing said biological information. To be more specific, when the measuring device 10 is used as the novel interface, the measuring device 10 can sufficiently follow the movement of the measurement object and thus can easily measure the voltage serving as input data, by merely pressing the electrode 12 against the region targeted for measurement. Moreover, the measuring device 10 facilitates fixation to the finger 2 without suffering from adverse effects on the skin of the finger 2. In this way, the measuring device 10 can measure the voltage accurately and thereby effectuate accurate data input. Therefore the measuring device 10 also achieves ease of use as an interface for daily usage. In this way, the measuring device 10 can meet the requirements for the interface. Needless to say, usage of the measuring device 10 is not limited to technological application of the detected brain waves or EMG. It is also possible to utilize the measuring device 10 for easily measuring the brain waves and the EMG for medical purposes.

Furthermore, regarding the fixing member 11, the contact surface 11a to contact the finger 2, which is the fixation object, is made of the nonconductive material. In other words, the fixing member 11 has the nonconductive contact surface 11a in contact with the fixation object. For this reason, in the measuring device 10, the fixing member 11 can electrically insulate between the finger 2 and the electrode 12. Therefore, the electrode 12 can appropriately measure only the voltage on the skin 3, which is the measurement object, on the surface of the head, on the surface of the face, on the surface of the arm, or on the surface of the leg. In other words, the electrode 12 can measure only the necessary voltage without suffering from an adverse effect of an unnecessary voltage such as a voltage generated on the finger 2. Moreover, the measuring device 10 can transmit the measured voltage externally by use of the lead wire 13.

Therefore, the external device such as the amplifier or the computing unit can detect only the electric potential variation of the measurement object from the voltage, and detect only the necessary biological information of the measurement object such as the EMG or the brain waves from the electric potential variation. That is, the external device such as the amplifier or the computing unit can detect only the necessary biological information without suffering from unnecessary signals such as the EMG of the finger 2. Moreover, the amplifier or the computing unit can apply the biological information to many purposes such as the speech recognition processing or the recognition processing for a motion or an action. In this way, the measuring device 10 is significantly different from a device including an electrode, which is electrically connected to a human body, which is the fixation object, and gives electrical stimulation to the fixed human body, for the purpose of a brain evoked potentials, or evoked electromyography.

Moreover, the measuring device 10 includes the annular fixing member 11, which includes the annular portion configured to fit to the finger 2, and the single electrode 12. Accordingly, it is possible to flexibly deal with the variation in the number of measurement places only by fitting as many of the measuring devices 10 as the number of the measurement places. In addition, the measuring devices 10 can easily change the distance between the electrodes 12 for measurement by merely changing the positions of the fixing members 11 fitted to the finger 2. For example, the distance between the electrodes 12 can be changed by fixing two measuring devices 10 to one finger 2 or by fixing the two measuring devices 10 separately to two fingers 2. It is also possible to change the distance between the electrodes 12 easily and flexibly by changing the fingers to which the measuring devices 10 are fitted, by way of fitting the measuring devices 10 separately to a thumb and a little finger, or a middle finger and a ring finger; or by spreading or closing the fingers 2 to which the measuring devices 10 are fitted. For this reason, the external device such as the amplifier or the computing unit can detect the electric potential difference between the separated electrodes 12, i.e. between two separated points.

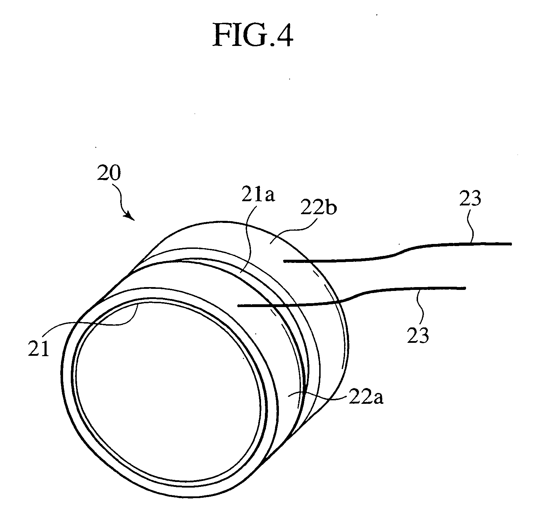

As shown in Fig. 4, a measuring device 20 comprises a fixing member 21, electrodes 22a and 22b, and lead wires 23. Regarding the measuring device 20, the fixation object is also the finger of the human body and the measurement object is the skin on the surface of the head, on the surface of the face, on the surface of the arm, or on the surface of the leg.

The fixing member 21 is formed into an annular shape to be a ring shape, which can be fitted to the finger, which is the fixation object. In other words, the fixing member 11 includes an annular portion configured to fit to the fixation object. The two electrodes 22a and 22b are mounted on the opposite side of the single fixing member 21 to a contact surface with the finger, i.e. on the outer side of the fixing member 21, with an interval equivalent to a gap 21a between the electrode 22a and the electrode 22b. Accordingly, the fixing member 21 has a width, which is sufficient for mounting the two electrodes 22a and 22b with the interval equivalent to the gap 21a therebetween while preventing contact between the finger and the electrodes 22a and 22b. In the measuring device 20, the width of the fixing member 21 is equal to the sum of the widths of the two electrodes 22a and 22b and the width of the gap 21a.

In the measuring device 20, the outer side of the ring of the fixing member 21 is made flat. When the electrodes 22a and 22b are mounted on the outer side of the fixing member 21, the gap 21a is formed thereon, which is a concave portion formed between the electrodes 22a and 22b. Fabrication is facilitated in accordance with the above-described manner because the fixing member 21 can be simply formed into an annular shape. In this case, the fixing member 21 is made of a nonconductive material of a solid type or a sheet type, so that the electrodes 22a and 22b can be fixed to the fixing member 21 while providing the gap 21a therebetween.

Here, it is also possible to form a convex portion in the nonconductive material for filling the gap 21a on the outer side of the ring of the fixing member 21. In this way, it is possible to provide the interval between the two electrodes 22a and 22b and to partition the two electrodes 22a and 22b with the convex portion made of the nonconductive material. In this case, the fixing member 21 can also be made of the nonconductive material of a solid type or a sheet type. Alternatively, it is also possible to fix the electrodes 22a and 22b to both ends of a member, which forms the convex portion, and to use a thin film type material formed by coating the inner surfaces of the rings of the electrodes 22a and 22b as the fixing member 21.

The fixing member 21 is made of the nonconductive material. Accordingly, the contact surface of the fixing member 21 to contact the finger, which is the fixation object, is made of the nonconductive material. Furthermore, a setting surface on which the electrodes 22a and 22b are mounted and which is the opposite side of the fixing member 21 to the contact surface with the finger, which is the fixation object, is also made of the nonconductive material. In other words, the fixing member 21 has a nonconductive opposite surface to the contact surface with the fixation object. In this way, the finger and the electrodes 22a and 22b are electrically insulated by the fixing member 21, and the electrodes 22a and 22b are also electrically insulated. Other aspects of the fixing member 21 are substantially similar to the fixing member 11 shown in Fig. 1.

The two electrodes 22a and 22b are mounted on the opposite side of the single fixing member 21 to the contact surface with the finger. The electrodes 22a and 22b are formed into annular shapes having the width for covering part of the outer side of the ring of the fixing member 21. Moreover, the electrodes 22a and 22b are mounted on the outer side of the fixing member 21 with the interval equivalent to the gap 21a between the electrodes 22a and 22b. The electrodes 22a and 22b are mounted so that the sum of the widths of the electrodes 22a and 22b and the width of the gap 21a is equal to the width of the fixing member 21, and so that one end of each of the electrodes 22a and 22b on the opposite side to the gap 21a agrees with both ends of the fixing member 21 when the measuring device 20 is viewed from above. Alternatively, it is also possible to mount the electrodes 22a and 22b so that an end face of the electrode 22a on the opposite side to the gap 21a is located inside one end face of the fixing member 21 in the width direction and at the same time, an end face of the electrode 22b on the opposite side to the gap 21a is located inside the other end face of the fixing member 21 in the width direction. Accordingly, the electrodes 22a and 22b are prevented from contacting the finger when the measuring device 20 is fixed to the finger.

Other aspects of the electrodes 22a and 22b are substantially similar to the electrode 12 shown in Fig. 1. One end of each lead wire 23 is connected to part of a measurement surface where the electrode 22a or 22b contacts the measurement object and measures voltage. Other aspects of the lead wires 23 are substantially similar to the lead wire 13 shown in Fig. 1.

Upon measurement of voltage by use of the measuring device 20, the subject puts the single measurement device 20 on one finger. Next, the subject presses the electrodes 22a and 22b against a region targeted for measurement, such as the skin on the surface of the head, of the face, of the arm, or of the leg to make contact. In this way, voltage is induced between the two electrodes 22a and 22b, and the electrodes 22a and 22b thereby measure the voltage. The lead wires 23 transmit the measured voltage and deliver the voltage to the external device such as the amplifier, the computing unit, or the output device. In this way, the measured voltage is transmitted externally thorough the lead wires 23. The amplifier or the computing unit detects the electric potential variation between the two electrodes 22a and 22b, and detects the biological information such as brain waves or EMG from the electric potential variation. Then, the computing unit performs the speech recognition processing or the recognition processing for a motion or an action by use of the biological information. The output device outputs the measured voltage and the detected signals as measured values.

According to the measuring device 20, the two electrodes 22a and 22b are mounted on the single fixing member 21 with the interval equivalent to the gap 21a therebetween. Moreover, since the fixing member 21 is made of the nonconductive material, the setting surface which is the opposite side of the fixing member 21 to the contact surface with the finger, which is the fixation object, and on which the electrodes 22a and 22b are mounted, is made of the nonconductive material. In other words, the fixing member 21 has the nonconductive opposite surface to the contact surface with the fixation object. Therefore, the measuring device 20 can connect the two electrodes 22a and 22b to the measurement object in a state where the two electrodes 22a and 22b are electrically insulated by fixing member 21 made of the nonconductive material, while holding a constant interval therebetween. In this way, the measuring device 20 can measure a difference in voltage between the electrodes 22a and 22b and thereby measure the electric potential variation between both electrodes 22a and 22b while holding a constant interval therebetween. Therefore, the measuring device 20 is effective when the voltage is expected to be measured under a constant measurement condition, for example. Moreover, since the plurality of electrodes 22a and 22b can be mounted on the single fixing member 21, the single measuring device 20 can perform the measurement.

Here, regarding the measuring device 20, the two electrodes 22a and 22b are mounted on the single fixing member 21. However, the number of the electrodes to be mounted on the single fixing member 21 is not limited. It is possible to mount three or more electrodes on the single fixing member 21 if sufficient intervals are provided severally between the electrodes. In this case, the single measuring device can measure a plurality of places.

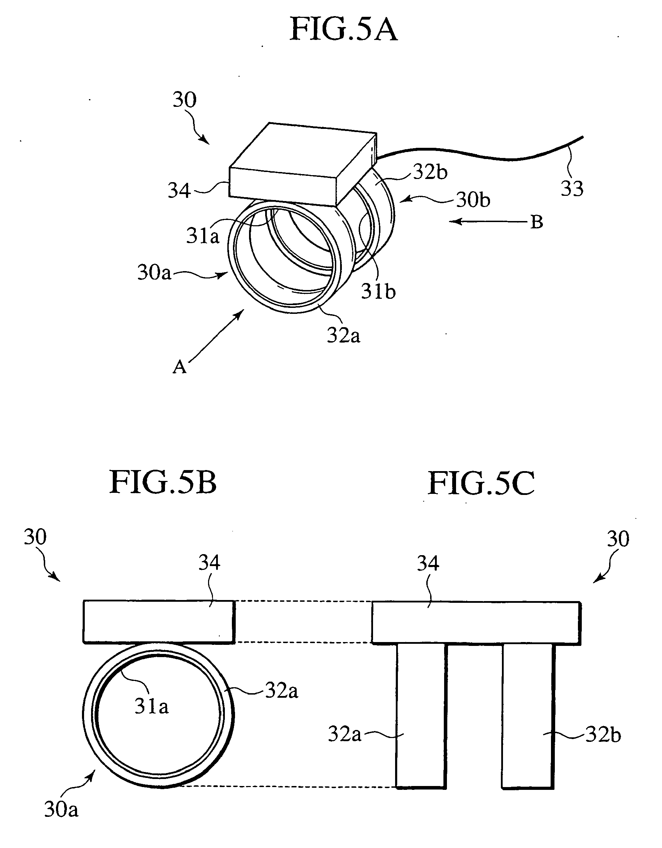

Fig. 5A is a perspective view of an active electrode-type measuring device 30 which is helpful for understanding the present invention. Fig. 5B is a front view from the direction indicated with the arrow A shown in Fig. 5A. Fig. 5C is a side view from the direction indicated with the arrow B shown in Fig. 5A.

As shown in Fig. 5A, the active electrode-type measuring device 30 comprises two main units 30a and 30b, a lead wire 33, and an amplifier 34. Regarding the active electrode-type measuring device 30, the fixation object is also the finger of the human body and the measurement object is the skin on the surface of the head, on the surface of the face, on the surface of the arm, or on the surface of the leg. The active electrode-type measuring device 30 is a measuring device, which includes the amplifier 34 configured to amplify voltage measured by electrodes 32a and 32b.

Each of the main units 30a and 30b includes a fixing member 31a or 31b, and the electrode 32a or 32b, respectively. Each of the fixing members 31a and 31b, and each of the electrodes 32a and 32b, are substantially similar to the fixing member 11 and the electrode 12 shown in Fig. 1, respectively. Accordingly, each of the main units 30a and 30b has a similar constitution to the measuring device 10 shown in Fig. 1, except that the lead wire is not mounted on the measurement surface of the electrode 32a or 32b.

The amplifier 34 amplifies the voltage measured by the electrodes 32a and 32b. As shown in Figs. 5A to 5C, the amplifier 34 is mounted on upper parts of the main units 30a and 30b, which are arranged in parallel to have side faces thereof face each other while holding an interval, so as to contact the electrodes 32a and 32b. Measured voltage acquiring portions configured to acquire the voltage from the electrodes 32a and 32b are mounted on part of the contact surface of the amplifier 34, which contacts the electrodes 32a and 32b. For example, each of the measured voltage acquiring portions is made of a conductive material, and is electrically connected to the respective electrodes 32a and 32b. Moreover, the amplifier 34 acquires the voltage from the electrodes 32a and 32b through the measured voltage acquiring portions.

As described above, the amplifier 34 is mounted so as to contact the electrodes 32a and 32b, and the measured voltage acquiring portions are mounted on part of the contact surface thereof. Accordingly, the amplifier 34 can acquire the voltage measured by the electrodes 32a and 32b without using a lead wire. Since the amplifier 34 is mounted so as to contact the electrodes 32a and 32b, the amplifier 34 can amplify the measured voltage in the closest vicinity of the electrodes 32a and 32b. For this reason, it is preferable that the amplifier 34 is mounted so as to contact the electrodes 32a and 32b. Here, parts on the surface of the amplifier 34 other than the measured voltage acquiring portions are made of nonconductive material.

Even if the amplifier 34 does not contact the electrodes 32a and 32b, it is still preferable that the amplifier 34 is mounted as closely as possible to the electrodes 32a and 32b. For example, when the electrodes 32a and 32b are fixed to the finger, the amplifier 34 may be placed within the range from the finger to the wrist. To be more specific, it is preferable that the distance between the amplifier 34 and the electrodes 32a and 32b is set to about 20 cm. When the amplifier 34 is fixed to the wrist, it is possible to use a wrist watch-type amplifier as the amplifier 34, which is fixed to the wrist, like a wristwatch. When the amplifier 34 is mounted separately from the electrodes 32a and 32b without any contact as described above, the amplifier 34 acquires the voltage measured by the electrodes 32a and 32b from the electrodes 32a and 32b by use of another lead wire, or a shielded cable which can prevent noises from mixing into the measured voltage from the outside.

The amplifier 34 includes a lead wire input portion configured to input the amplified voltage to the lead wire 33. The lead wire 33 transmits externally the voltage measured by the electrodes 32a and 32b in the state after amplification by the amplifier 34. One end of the lead wire 33 is connected to the lead wire input portion of the amplifier 34. The other end of the lead wire 33 is connected to the external device such as the computing unit or the output device. The computing unit detects signals from the voltage, which have been measured by the electrodes 32a and 32b and amplified by the amplifier 34. Using the signals, the computing unit can process the speech recognition processing or the recognition processing for a motion or an action. The output device outputs the voltage amplified by the amplifier 34 and the signals detected from the voltage as measured values. The lead wire 33 transmits the voltage amplified by the amplifier 34 and delivers the voltage to the external device.

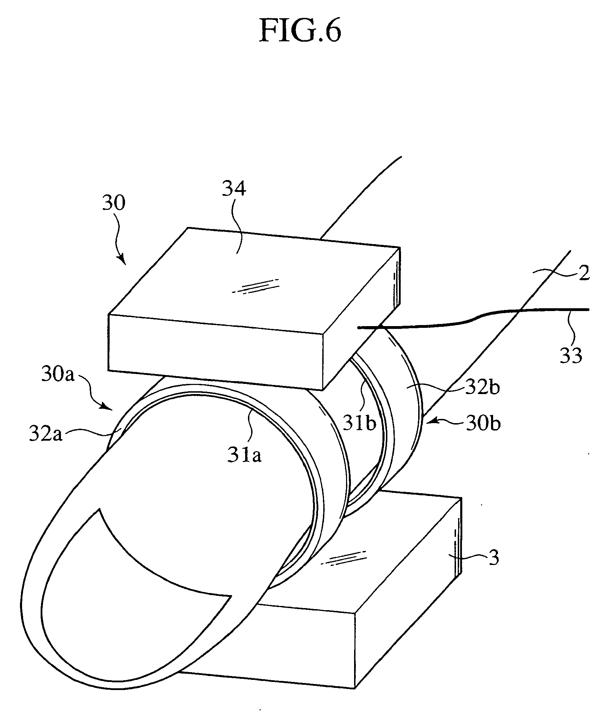

Next, description will be made regarding a method of measuring voltage by use of the active electrode-type measuring device 30. As shown in Fig. 6, the subject puts the fixing members 31a and 31b of the single active electrode-type measurement device 30 on one finger 2. Next, the subject presses the electrodes 32a and 32b against a region targeted for measurement, such as the skin 3 on the surface of the head, of the face, of the arm, or of the leg to make contact. In this way, voltage is induced between the two electrodes 32a and 32b, and the electrodes 32a and 32b thereby measure the voltage.

The amplifier 34 acquires the measured voltage from the electrodes 32a and 32b through the measured voltage acquiring portions mounted on part of the contact surface with the electrodes 32a and 32b. Then, the amplifier 34 amplifies the voltage and inputs the amplified voltage to the lead wire 33 through the lead wire input portion. The lead wire 33 transmits the amplified voltage acquired from the amplifier 34 and delivers the voltage to the external device such as the computing unit or the output device. Accordingly, the voltage thus measured by the electrodes 32a and 32b and amplified by the amplifier 34 is delivered externally through the lead wire 33. The computing unit detects the electric potential variation between the two electrodes 32a and 32b, and detects biological information such as brain waves or EMG from the electric potential variation.

According to the active electrode-type measuring device 30, which comprises the amplifier 34 configured to amplify the voltage measured by the electrodes 32a and 32b, the measured voltage is amplified by the amplifier 34. For this reason, the active electrode-type measuring device 30 can reduces noises, which mix into the measured voltage from the outside. In particular, since the amplifier 34 is mounted so as to contact the electrodes 32a and 32b in the active electrode-type measuring device 30, it is possible to amplify the measured voltage in the closest vicinity of the electrodes 32a and 32b. As a result, the active electrode-type measuring device 30 can further reduce noises, which mix into the measured voltage from the outside.

Moreover, the amplifier 34 is mounted on the upper parts of the main units 30a and 30b, which are arranged in parallel to have the side faces thereof face each other while holding the interval, so as to contact the electrodes 32a and 32b. Accordingly, the active electrode-type measuring device 30 can connect the plurality of electrodes 32a and 32b to the measurement object while holding the interval through the amplifier 34. Therefore, the active electrode-type measuring device 30 can measure the electric potential variation between the two electrodes 32a and 32b while holding a constant interval therebetween. Furthermore, since the plurality of electrodes 32a and 32b can be connected to the amplifier 34, it is possible to measure a plurality of places by use of the single active electrode-type measuring device 30. Here, regarding the active electrode-type measuring device 30, the two main units 30a and 30b are mounted on the single amplifier 34. However, the number of the main units to be mounted on the single amplifier 34 is not limited. It is possible to mount three or more main units on the single amplifier 34 if sufficient intervals are provided severally between the electrodes.

Moreover, in an embodiment of the present invention a plurality of electrodes is mounted on the single fixing member, similar to the measuring device 20 shown in Fig. 4. That is, in an embodiment, the fixing member 21 and the electrodes 22a and 22b shown in Fig. 4 constitute the main unit, and the amplifier 34 is mounted on an upper part of the main unit so as to contact the electrodes 22a and 22b. Furthermore, it is also possible to incorporate preamplifiers into the electrodes 32a and 32b. In this way, it is possible to further prevent noises from mixing into the measured voltage.

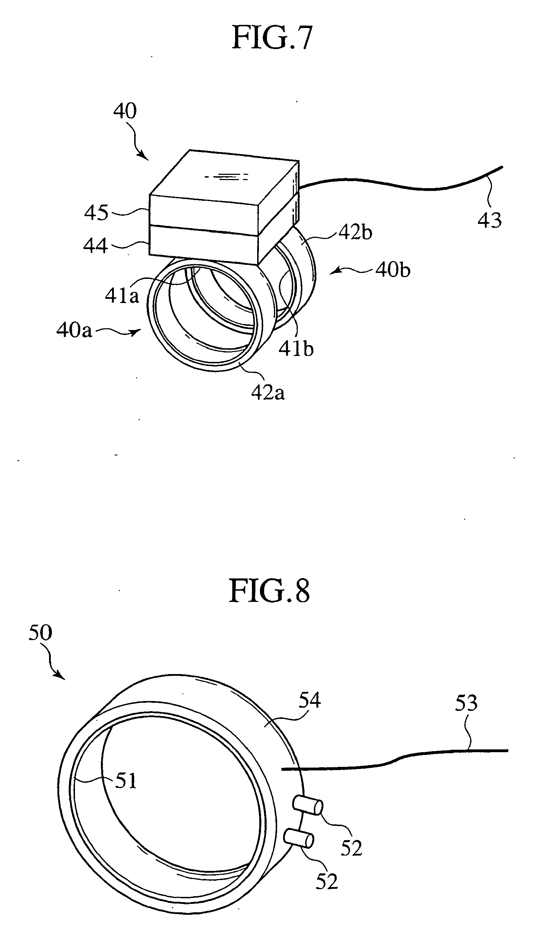

As shown in Fig. 7, an active electrode-type measuring device 40, which is helpful for understanding the present invention, comprises two main units 40a and 40b, a lead wire 43, an amplifier 44, and a computing unit 45. Regarding the active electrode-type measuring device 40, the fixation object is also the finger of the human body and the measurement object is the skin on the surface of the head, on the surface of the face, on the surface of the arm, or on the surface of the leg.

Each of the main units 40a and 40b includes a fixing member 41a and 41b respectively, and an electrode 42a and 42b respectively. Each of the main units 40a and 40b is substantially similar to the main unit 30a or 30b shown in Figs. 5A to 5C. The computing unit 45 is mounted on the amplifier 44 so as to contact an upper part thereof. A computing unit input portion configured to input amplified voltage to the computing unit 45 is mounted on part of a contact surface of the amplifier 44 with the computing unit 45. For example, the computing unit input portion is made of a conductive material and is electrically connected to part of a contact surface of the computing unit 45, which contacts the amplifier 44. Other aspects of the amplifier 44 are substantially similar to the amplifier 34 shown in Figs. 5A to 5C.

The computing unit 45 processes by use of the signals based on the voltage measured by the electrodes 42a and 42b. As to the processing by use of the signals based on the voltage measured by the electrodes 42a and 42b, the computing unit 45 performs the speech recognition processing for recognizing a voice by use of the signals and recognition processing for recognizing a motion or an action by use of the signals, for example. The computing unit 45 is mounted on the upper part of the amplifier 44 so that an upper face of the amplifier 44 and a lower face of the computing unit 45 contact each other. An amplified voltage acquiring portion configured to acquire the amplified voltage from the amplifier 44 is mounted on part of the contact surface of the computing unit 45 which contacts the amplifier 44. For example, the amplified voltage acquiring portion is made of a conductive material, and is electrically connected to the computing unit input portion mounted on the contact surface of the amplifier 44, which contacts the computing unit 45. Moreover, the computing unit 45 acquires the voltage after amplification from the amplifier 44 through the amplified voltage acquiring portion.

As described above, the computing unit 45 is mounted so as to contact the amplifier 44, and the amplified voltage, acquiring portion is mounted on part of the contact surface of the computing unit 45, which contacts the amplifier 44. Accordingly, the computing unit 45 can acquire the amplified voltage from the amplifier 44 without using lead wires but by electrically connecting the amplified voltage acquiring portion to the computing unit input portion of the amplifier 44. Moreover, the computing unit 45 is mounted so as to contact the amplifier 44, which is mounted so as to contact the electrodes 42a and 42b. Therefore, the computing unit 45 is mounted in the vicinity of the electrodes 42a and 42b. In this way, since the computing unit 45 is mounted in the vicinity of the electrodes 42a and 42b so as to contact the amplifier 44, it is possible to shorten the distance of transmission of the voltage from the electrodes 42a and 42b and the amplifier 44 to the computing unit 45. For this reason, the active electrode-type measuring device 40 can suppress the mixture of noises from the outside into the voltage or suppress attenuation of the amplified voltage. Moreover, it is also possible to downsize the entire active electrode-type measuring device 40. Therefore, it is preferable that the computing unit 45 is mounted so as to contact the amplifier 44, and in the vicinity of the electrodes 42a and 42b. Here, parts on the surface of the computing unit 45 other than the amplified voltage acquiring portion is made of nonconductive material.

Even if the computing unit 45 does not contact the amplifier 44, it is still preferable that the computing unit 45 is mounted as closely as possible to the amplifier 44. To be more specific, it is preferable that the distance between the computing unit 45 and the amplifier 44 is set to about 20 cm. In this case, the computing unit 45 can acquire the amplified voltage from the amplifier 44 by use of another lead wire or a shielded cable.

The computing unit 45 detects the electric potential variation between the two electrodes 42a and 42b based on the amplified voltage thus acquired. The computing unit 45 detects biological information such as brain waves or EMG from the electric potential variation. Then, the computing unit 45 performs the speech recognition processing or the recognition processing for a motion or an action by use of the detected biological information. The computing unit 45 includes a recognition result input portion configured to input the result of speech recognition or the result of recognition for the motion or the action to the lead wire 43.

The speech recognition processing includes processing for recognizing five Japanese vowels (/a/, /i/, /u/, /e/, /o/) by passing the detected EMG through a bandpass filter and counting the number of intersections of a threshold value, for example. The speech recognition processing also includes processing for recognizing voices including vowels and consonants by processing the detected EMG using a neutral network or a hidden Markov model (HMM).

The neutral network has a structure as described below. A group of elements, which is a set of nonlinear elements having input portions and output portions for parameters are hierarchically arranged from upstream toward downstream. Then, between two adjacent element groups, the input portions of the upstream nonlinear elements and the output portions of the downstream nonlinear elements are mutually connected. The neutral network may be a three-layer fully-connected neutral network, for example.

The processing using this neutral network is performed as described below. Firstly, the computing unit 45 divides the detected EMG into certain time periods. The computing unit 45 performs processing such as spectrum analysis, root-mean-square (RMS) processing, average-rectified-value (ARV) processing, or integrated electromyography (IEMG) for the divided EMG, and thereby calculates parameters based on the EMG (hereinafter such parameters will be referred to as "myoelectric signal parameters " ). Thereafter, the computing unit 45 recognizes a voice by inputting the myoelectric signal parameters to the input portions of the nonlinear elements constituting the neutral network and then making the output portions of the nonlinear elements output vowels or consonants corresponding to the myoelectric signal parameters.

The lead wire 43 transmits the voltage measured by the electrodes 42a and 42b externally, in the state after amplification by the amplifier 44 and after detection of the biological information such as the brain waves or the EMG from the amplified voltage and the subsequent processing such as the speech recognition processing or the recognition processing for the motion or the action being performed by the computing unit 45. One end of the lead wire 43 is attached to the recognition result input portion of the computing unit 45. The other end of the lead wire 43 is connected to an external device, such as an output device for outputting the result of speech recognition or the result of recognition for the motion or the action, or a device for utilizing the result of speech recognition or the result of recognition for the motion or the action. Moreover, the lead wire 43 transmits the voltage measured by the electrodes 42a and42b and delivers the voltage to the external device either as the result of speech recognition after performing the speech recognition processing or as the result of recognition for the motion or the action by use of the biological information based on the voltage.

Upon measurement of voltage by use of the active electrode-type measuring device 40, the subject puts the single active electrode-type measurement device 40 on one finger. Next, the subject presses the electrodes 42a and 42b against a region targeted for measurement, such as the skin on the surface of the head, on the surface of the face, on the surface of the arm, or on the surface of the leg to make contact. In this way, voltage is induced between the two electrodes 42a and 42b, and the electrodes 42a and 42b thereby measure the voltage.

The amplifier 44 acquires the voltage measured by the electrodes 42a and 42b through the measured voltage acquiring portion. Then, the amplifier 44 amplifies the voltage, and inputs the amplified voltage to the computing unit 45 from the computing unit input portion. The computing unit 45 acquires the amplified voltage from the amplifier 44 through the amplified voltage acquiring portion. The computing unit 45 detects the electric potential variation between the two electrodes 42a and 42b based on the acquired voltage after amplification. The computing unit 45 detects the biological information such as the brain waves or the EMG from the electric potential variation. Thereafter, the computing unit 45 performs the speech recognition processing or the recognition processing for the motion or the action by use of the detected biological information. Lastly, the computing unit 45 inputs the result of speech recognition or the result of recognition for the motion or the action to the lead wire 43 from the recognition result input portion. Then, the lead wire 43 transmits the result of speech recognition or the result of recognition for the motion or the action and delivers the result to the external device. In this way, the result of speech recognition or the result of recognition for the motion or the action using the biological information based on the voltage detected by the electrodes 42a and 42b and amplified by the amplifier 44 is delivered to the external device through the lead wire 43.

The active electrode-type measuring device 40 comprises the computing unit 45 configured to process by use of the signals based on the voltage measured by the electrodes 42a and 42b and amplified by the amplifier 44. Accordingly, the active electrode-type measuring device 40 can process by use of the signals based on the measured voltage. For example, the active electrode-type measuring device 40 can perform the speech recognition processing or the recognition processing for the motion or the action by use of the biological information based on the measured voltage. Therefore, instead of transmitting the measured voltage unprocessed, the active electrode-type measuring device 40 can transmit the measured voltage to the external device in the state after detection of the signals such as the biological information based on the voltage, and after performing the processing such as the speech recognition processing or the recognition processing for the motion or the action. That is, the active electrode-type measuring device 40 can complete the speech recognition processing or the recognition processing for the motion or the action by itself.

Here, it is also possible to incorporate preamplifiers into the electrodes 42a and 42b. In this way, it is possible to further prevent noises from mixing into the measured voltage. Moreover, although the active electrode-type measuring device 40 is formed as the active electrode type including the amplifier 44, it is also possible to form a passive electrode-type measuring device without provision of the amplifier 44. When the amplifier 44 is not mounted thereon, the computing unit 45 can acquire the voltage measured by the electrodes 42a and 42b by direct connection with the electrodes 42a and 42b. In this case, it is preferable that the computing unit 45 is mounted so as to contact the electrodes 42a and 42b.

Alternatively, even if the computing unit 45 does not contact the electrodes 42a and 42b, it is still preferable that the computing unit 45 is mounted as closely as possible to the electrodes 42a and 42b. For example, when the electrodes 42a and 42b are fixed to the finger, the computing unit 45 may be placed within the range from the finger to the wrist. To be more specific, it is preferable that the distance between the computing unit 45 and the electrodes 42a and 42b is set to about 20 cm. When the computing unit 45 is fixed to the wrist, it is possible to use a wrist watch-type computing unit as the computing unit 45, which is fixed to the wrist like a wrist watch. When the computing unit 45 is mounted separately from the electrodes 42a and 42b without any contact as described above, the computing unit 45 acquires the voltage measured by the electrodes 42a and 42b from the electrodes 42a and 42b by use of another lead wire or a shielded cable.

As shown in Fig. 8, an active electrode-type measuring device 50 not forming an embodiment of the present invention comprises a fixing member 51, electrodes 52, a lead wire 53, and an amplifier 54. The active electrode-type measuring device 50 has a two-layer structure formed by the fixing member 51 and the amplifier 54. Regarding the active electrode-type measuring device 50, the fixation object is also the finger of the human body and the measurement object is the skin on the surface of the head, on the surface of the face, on the surface of the arm, or on the surface of the leg.

The fixing member 51 constitutes the inner side of the two-layer structure formed by the fixing member 51 and the amplifier 54. The amplifier 54 is mounted on the opposite side of the fixing member 51 to a contact surface with the fixation object, that is, on an outer side of the ring of the fixing member 51. Other aspects of the fixing member 51 are substantially similar to the fixing member 11 shown in Fig. 1. The amplifier 54 is mounted on the opposite side of the fixing member 51 to the contact surface with the finger, which is the fixation object. To be more specific, the amplifier 54 is formed into an annular shape for covering the outer side of the ring of the fixing member 51 and is mounted on the opposite side to the contact surface with the finger. In this way, the amplifier 54 constitutes the outer side of the two-layer structure formed by the fixing member 51 and the amplifier 54.

If the fixing member 51 is of a solid type, it is possible to form the ring shape of the two-layer structure including the fixing member 51 and the amplifier 54 by fitting the fixing member 51 into the inner side of the ring of the amplifier 54. If the fixing member 51 is of a sheet type or of a thin-film type, it is possible to form the ring shape of the two-layer structure including the fixing member 51 and the amplifier 54 by attaching the fixing member 51 to the inner surface of the ring of the amplifier 54 or by coating the inner surface with the material for the fixing member 51 to form the fixing member 51 of the thin-film type.

Moreover, the amplifier 54 holds a plurality of electrodes 52. To be more specific, the plurality of electrodes 52 are mounted on the opposite side of the amplifier 54 to the contact surface with the fixing member 51, that is, on an outer side of the ring of the amplifier 54. Other aspects of the amplifier 54 are substantially similar to the amplifier 34 shown in Figs. 5A to 5C.

The electrodes 52 are mounted on the opposite side of the amplifier 54 to the contact surface with the fixing member 51, that is, on the outer side of the ring of the amplifier 54. In other words, the electrodes 52 are mounted on the opposite side to the contact surface of the fixing member 51, which contacts the fixation object, through the amplifier 54. The electrodes 52 are formed into columnar shapes. In addition to a column shape, a prism shape, a cone shape, or a pyramid shape can be also used for the electrode 52.

One end of the electrode 52 contacts the outer surface of the ring of the amplifier 54 and is thereby fixed to the amplifier 54. Moreover, a tip portion of the electrode 52, which is the other end of the electrode 52, contacts the measurement object and serves as a measurement surface for measuring electric potential. The two electrodes 52 are mounted on the amplifier 54 with an interval therebetween. Here, the number of the electrodes 52 to be mounted on the amplifier 54 is not limited. It is possible to mount more than two electrodes 52 if sufficient intervals are provided severally therebetween. Other aspects of the electrode 52 are substantially similar to the electrode 12 shown in Fig. 1. The lead wire 53 is substantially similar to the lead wire 33 shown in Figs. 5A to 5C.

Upon measurement of voltage by use of the active electrode-type measuring device 50, the subject puts the single active electrode-type measurement device 50 on one finger. Next, the subject presses the tip portions, which are the measurement surfaces, of the electrodes 52 against a region targeted for measurement, such as the skin on the surface of the head, on the surface of the face, on the surface of the arm, or on the surface of the leg to make contact. In this way, voltage is induced between the two electrodes 52, and the electrodes 52 thereby measure the voltage. Thereafter, the amplifier 54 and the lead wire 53 operate substantially similarly to the active electrode-type measuring device 30 shown in Figs. 5A to 5C.

According to the active electrode-type measuring device 50, the electrodes 52 are formed into the columnar shapes, and the tip portions of the electrodes 52 constitute the measurement surfaces for contacting the measurement object and thereby measuring the electric potential. Accordingly, the active electrode-type measuring device 50 can reduce the contact area of the measurement surface of the electrode 52 and the skin on the surface of the head, on the surface of the face, on the surface of the arm, or on the surface of the leg, which is the measurement object. Therefore, the electrode 52 can measure variation in voltage attributable to delicate movement of a muscle or the skin of the head, of the face, of the arm, or of the leg. Hence, the active electrode-type measuring device 50 can measure while following the delicate movement of the measurement object more accurately.

Moreover, the electrodes 52 are mounted on the amplifier 54 with an interval therebetween. Therefore, the active electrode-type measuring device 50 can measure the electric potential variation between the two electrodes 52 while providing a constant interval therebetween. Furthermore, since the plurality of electrodes 52 can be mounted on the single amplifier 54, it is possible to measure a plurality of places by use of the single active electrode-type measuring device 50.

Moreover, although the active electrode-type measuring device 50 is formed as the active electrode type including the amplifier 54, it is also possible to form a passive electrode-type measuring device without provision of the amplifier 54. In this case, the fixing member 51 holds the plurality of electrodes 52, similar to the measuring device 10 shown in Fig. 1. That is, the electrodes 52 are mounted on the opposite side of the fixing member 51 to the contact surface with the fixation object, i.e. on the outer side of the ring of the fixing member 51. Meanwhile, one end of the lead wire 53 is connected to the electrode 52. Furthermore, in this case, it is possible to prevent noises from mixing into the measured voltage by incorporating preamplifiers into the electrodes 52.

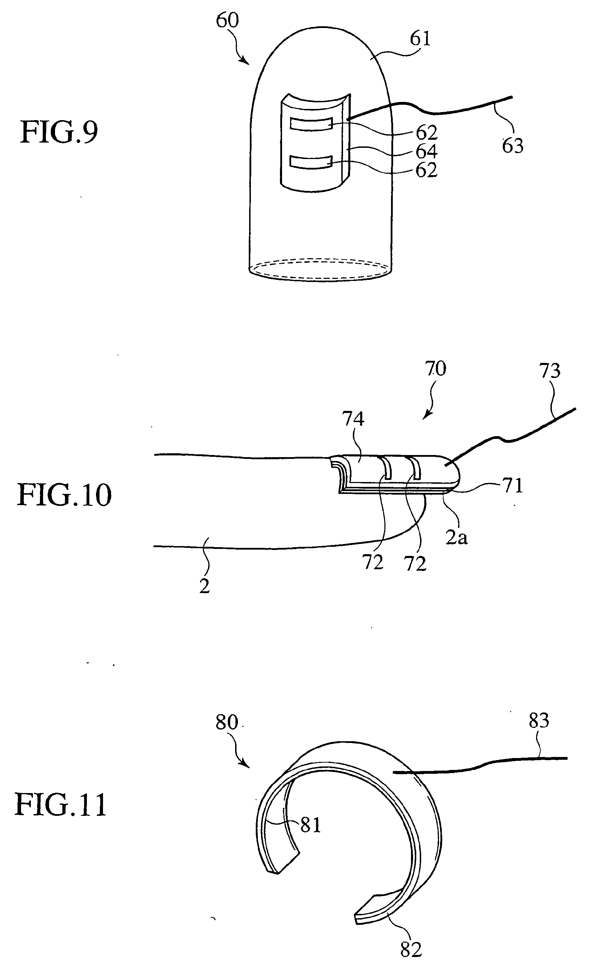

A background example which does not form an embodiment of the present invention will be described with reference to the accompanying drawing. As shown in Fig. 9, an active electrode-type measuring device 60 includes a fixing member 61, electrodes 62, a lead wire 63, and an amplifier 64. Regarding the active electrode-type measuring device 60, the fixation object is also the finger of the human body and the measurement object is the skin on the surface of the head, on the surface of the face, on the surface of the arm, or on the surface of the leg.

The fixing member 61 comprises a concave portion, which is fitted to the finger, which is the fixation object. A tip of the finger, which is the fixation object, is inserted from an opening of the concave portion of the fixing member 61, and the concave portion is thereby fitted to the finger, which is the fixation object. Therefore, an inner surface of the concave portion of the fixing member 61 constitutes a contact surface for contacting the fixation object when fixed to the fixation object. Moreover, the fixing member 61 holds the amplifier 64. To be more specific, the amplifier 64 is mounted on the opposite side of the fixing member 61 to the contact surface with the fixation object, that is, on part of an outer side of the concave portion of the fixing member 61. For this reason, the fixing member 61 of a solid type or of a sheet type is used. Other aspects of the fixing member 61 are substantially similar to the fixing member 11 shown in Fig. 1.

The amplifier 64 is mounted on the opposite side of the fixing member 61 to the contact surface with the finger, which is the fixation object. To be more specific, the amplifier 64 is mounted on the outer side of the concave portion of the fixing member 61. Moreover, the amplifier 64 holds a plurality of the electrodes 62. To be more specific, the plurality of electrodes 62 are mounted on the opposite side of the amplifier 64 to the contact surface with the fixing member 61, that is, on an outer side of the amplifier 64. Other aspects of the amplifier 64 are substantially similar to the amplifier 34 shown in Figs. 5A to 5C.

The electrodes 62 are mounted on the opposite side of the amplifier 64 to the contact surface with the fixing member 61, that is, on the outer side of the amplifier 64. In other words, the electrodes 62 are mounted on the opposite side of the fixing member 61 to the contact surface with the fixation object, through the amplifier 64. The electrodes 52 are formed into band shapes. The shape of the electrode 62 is not only limited to the band shape, and a column shape, a prism shape, a cone shape, and a pyramid shape are also applicable. One surface of the electrode 62 contacts the outer surface of the amplifier 64 and is thereby fixed to the amplifier 64. Moreover, the other surface of the electrode 62 contacts the measurement object and constitutes a measurement surface for measuring electric potential. The two electrodes 62 are mounted on the amplifier 64 with an interval therebetween. Here, the number of the electrodes 62 to be mounted on the amplifier 64 is not limited. It is possible to mount more than two electrodes 64 if sufficient intervals are provided severally therebetween. Other aspects of the electrode 62 are substantially similar to the electrode 12 shown in Fig. 1. The lead wire 63 is substantially similar to the lead wire 33 shown in Figs. 5A to 5C.

Upon measurement of voltage by use of the active electrode-type measuring device 60, the subject inserts the finger from the opening of the concave portion of the fixing member 61 and thereby puts the single active electrode-type measurement device 60 on one finger. Next, the subject presses the two electrodes 62 against a region targeted for measurement, such as the skin on the surface of the head, on the surface of the face, on the surface of the arm, or on the surface of the leg to make contact. In this way, voltage is induced between the two electrodes 62, and the electrodes 62 thereby measure the voltage. Thereafter, the amplifier 64 and the lead wire 63 operate as substantially similarly to the active electrode-type measuring device 30 shown in Figs. 5A to 5C.

According to the active electrode-type measuring device 60, it is possible to fix the electrodes 62 to the finger without using tape but by merely fitting the concave portion included in the fixing member 61 to the finger, which is the fixation object. In addition, upon measurement, all that is required is to press the electrodes 62 against the measurement object. Accordingly, the electrode 62 themselves or a surrounding member of the electrode 62 does not need to be adhesive. As described above, the active electrode-type measuring device 60 does not need to be fixed to the fixation object or the measurement object with tape or an adhesive. Therefore, it is possible to suppress adverse effects on the fixation object or the measurement object, such as adverse effects on the finger, and the skin on the surface of the head, on the surface of the face, on the surface of the arm, or on the surface of the leg.

Moreover, since the concave portion of the fixing member 61 is fixed to the finger so as to cover the tip of the finger, it is possible to stably fix the electrodes 62 to the finger, and it is easy to press the electrodes 62 against the measurement object. For this reason, the degree of contact of the electrodes 62 with the measurement object can be easily adjusted, by merely adjusting the degree of the above-described pressure. As a result, the active electrode-type measuring device 60 can deal with the movement of muscles more flexibly.

Moreover, the electrodes 62 are mounted on the amplifier 64 with an interval therebetween. Therefore, the active electrode-type measuring device 60 can measure the electric potential variation between the two electrodes 62 while providing a constant interval therebetween. Furthermore, since the plurality of electrodes 62 can be mounted on the single amplifier 64, it is possible to measure a plurality of places by use of the single active electrode-type measuring device 60.

Although the active electrode-type measuring device 60 is formed as the active electrode type including the amplifier 64, it is also possible to form a passive electrode-type measuring device without provision of the amplifier 64. In this case, the fixing member 61 holds the plurality of electrodes 62, similar to the measuring device 10 shown in Fig. 1. That is, the electrodes 62 are mounted on the opposite side of the fixing member 61 to the contact surface with the fixation object, i.e. on the outer side of the concave portion of the fixing member 61. Meanwhile, one end of the lead wire 63 is connected to the electrode 62. Furthermore, in this case, it is possible to prevent noises from mixing into the measured voltage by incorporating preamplifiers into the electrodes 62.

As shown in Fig. 10, an active electrode-type measuring device 70, which does not form an embodiment of the present invention, comprises a fixing member 71, electrodes 72, a lead wire 73, and an amplifier 74. Regarding the active electrode-type measuring device 70, the fixation object is a nail 2a of the human body and the measurement object is the skin on the surface of the head, on the surface of the face, on the surface of the arm, or on the surface of the leg. The fixing member 71 and the amplifier 74 constitute a two-layer structure.

The fixing member 71 includes a curved portion, which fits along the nail 2a, which is the fixation object. The fixing member 71 is fixed to the nail 2a, which is the fixation object, by superposing the fixing member 71 on the nail 2a so that the curved portion and a curved surface of the nail 2a fit together. Therefore, an inner surface of the curved portion of the fixing member 71 serves as a contact surface for contacting the fixation object when fixed to the fixation object. Here, it is also possible to mount an adhesive attachment member on part of the contact surface of the fixing member 71 with the nail 2a or to impart adhesiveness to part of the contact surface of the fixing member 71 with the nail 2a. In this way, the fixing member 71 is fixed to the nail 2a more stably.

The fixing member 71 constitutes the lower part of the two-layer structure formed by the fixing member 71 and the amplifier 74. To be more specific, the amplifier 74 is mounted on the opposite side of the fixing member 71 to the contact surface with the fixation object, that is, on an outer side of the curved portion of the fixing member 71. Other aspects of the fixing member 71 are substantially similar to the fixing member 11 shown in Fig. 1.

The amplifier 74 is mounted on the opposite side of the fixing member 71 to the contact surface with the nail 2a, which is the fixation object. To be more specific, the amplifier 74 is formed into a curved shape so as to cover the outer side of the curved portion of the fixing member 71, and is mounted on the opposite side of the fixing member 71 to the contact surface with the nail 2a. Accordingly, the amplifier 74 constitutes the upper part of the two-layer structure formed by the fixing member 71 and the amplifier 74.

If the fixing member 71 is of a solid type, it is possible to form the two-layer structure including the fixing member 71 and the amplifier 74 by superposing the fixing member 71 and the amplifier 74. If the fixing member 71 is of a sheet type or of a thin-film type, it is possible to mount the fixing member 71 having the curved portion by attaching the fixing member 71 to the inner surface of the amplifier 74, or by coating the inner surface of the amplifier 74 with the material for the fixing member 71 to form the fixing member 71 of the thin-film type.

The amplifier 74 holds a plurality of the electrodes 72. To be more specific, the plurality of electrodes 72 are mounted on the opposite side of the amplifier 74 to the contact surface with the fixing member 71, that is, on an outer side of the amplifier 74. Other aspects of the amplifier 74 are substantially similar to the amplifier 34 shown in Figs. 5A to 5C. The electrode 72 is substantially similar to the electrode 62 shown in Fig. 9. Moreover, the lead wire 73 is substantially similar to the lead wire 33 shown in Figs. 5A to 5C.

Upon measurement of voltage by use of the active electrode - type measuring device 70, the subject superposes the fixing member 71 on the nail 2a so that the curved portion of the fixing member 71 and the curved surface of the nail 2a fit together, and thereby fixes the single active electrode-type measuring device 70 to the nail 2a. Next, the subject presses the two electrodes 72 against a region targeted for measurement, such as the skin on the surface of the head, on the surface of the face, on the surface of the arm, or on the surface of the leg to make contact. In this way, voltage is induced between the two electrodes 72, and the electrodes 72 thereby measure the voltage. Thereafter, the amplifier 74 and the lead wire 73 operate substantially similarly to the active electrode-type measuring device 30 shown in Figs. 5A to 5C.

According to the active electrode-type measuring device 70, even if the fixation object has a curved shape such as the nail 2a, it is possible to easily fix the electrodes 72 to the nail 2a because the curved portion of the fixing member 71 fits along the nail 2a, which is the fixation object. Moreover, the electrodes 72 are mounted on the amplifier 74 with an interval therebetween. Therefore, it is possible to measure the electric potential variation between the two electrodes 72 while providing a constant interval therebetween. Furthermore, since the plurality of electrodes 72 can be mounted on the single amplifier 74, it is possible to measure a plurality of places by use of the single active electrode-type measuring device 70.

Although the active electrode-type measuring device 70 is formed as the active electrode type including the amplifier 74, it is also possible to form a passive electrode-type measuring device without provision of the amplifier 74. In this case, the fixing member 71 holds the plurality of electrodes 72, similar to the measuring device 10 shown in Fig. 1. That is, the electrodes 72 are mounted on the opposite side of the fixing member 71 to the contact surface with the fixation object, i.e. on the outer side of the curved portion of the fixing member 71. Meanwhile, one end of the lead wire 73 is attached to the electrode 72. Furthermore, in this case, it is possible to prevent noises from mixing into the measured voltage by incorporating preamplifiers into the electrodes 72.

As shown in Fig. 11, a measuring device 80 comprises a fixing member 81, an electrode 82, and a lead wire 83. Regarding the measuring device 80, the fixation object is the finger or the wrist of the human body and the measurement object is the skin on the surface of the head, on the surface of the face, on the surface of the arm, or on the surface of the leg. The fixing member 81 and the electrode 82 constitute a two-layer structure.

The fixing member 81 includes a grasping portion configured to grasp the finger or the wrist, which is the fixation object. To be more specific, the fixing member 81 is formed into a C-shape by cutting part of an annular shape away. The fixing member 81 is fixed to the finger or the wrist by grasping the finger or the wrist with the C-shaped grasping portion. Therefore, an inner surface of the grasping portion of the fixing member 81 serves as a contact surface for contacting the fixation object when fixed to the fixation object. The fixing member 81 constitutes the inner side of the two-layer structure formed by the fixing member 81 and the electrode 82, and holds the electrode 82. To be more specific, the single electrode 82 is mounted on the opposite side of the single fixing member 81 to the contact surface with the fixation object, that is, on an outer side of the grasping portion of the single fixing member 81.

The electrode 82 is mounted on the opposite side of the fixing member 81 to the contact surface with the finger, which is the fixation object. To be more specific, the electrode 82 forms a C-shape for covering the outer side of the grasping portion of the fixing member 81, and is mounted on the opposite side of the fixing member 81 to the contact surface with the fixation object. Therefore, the electrode 82 constitutes the outer side of the two-layer structure formed by the fixing member 81 and the electrode 82. Meanwhile, the electrode 82 contacts the measurement object and thereby includes a measurement surface for measuring electric potential. The entire surface of the electrode 82 on the opposite side to a contact surface with the fixing member 81 serves as the measurement surface.

Other aspects of the fixing member 81 and the electrode 82 are substantially similar to the fixing member 11 and the electrode 12 shown in Fig. 1, respectively. Moreover, the lead wire 83 is substantially similar to the lead wire 13 shown in Fig. 1

Upon measurement of voltage by use of the measuring device 80, the subject fixes two measuring devices 80 on one finger or one wrist by grasping the finger or the wrist with the C-shaped grasping portions of the fixing members 81. Next, the subject presses the electrodes 82 against a region targeted for measurement, such as the skin on the surface of the head, on the surface of the face, on the surface of the arm, or on the surface of the leg to make contact. In this way, voltage is induced between the two electrodes 82, and the electrodes 82 thereby measure the voltage. Thereafter, the lead wire 83 operates substantially similarly to the measuring device 10 shown in Fig. 1.

According to the measuring device 80, it is possible to fix the electrode 82 to the finger or the wrist, which is the fixation object, easily and stably without using tape, by merely grasping the finger or the wrist, which is the fixation object, with the grasping portion of the fixing member 81. In addition, upon measurement, all that is required is to press the electrode 82 against the measurement object. Accordingly, the electrode 82 itself or a surrounding member of the electrode 82 does not need to be adhesive. As described above, the measuring device 80 does not need to be fixed to the fixation object or the measurement object with tape or an adhesive. Therefore, it is possible to suppress adverse effects on the fixation object or the measurement object, such as adverse effects on the finger, and the skin on the surface of the head, on the surface of the face, on the surface of the arm, or on the surface of the leg.

Moreover, since the measuring device 80 is fixed to the fixation object by merely grasping the fixation object with the grasping portion of the fixing member 81, the measuring device 80 can flexibly deal with the circumference of the finger or the wrist, and the like. Here, the shape of the grasping portion is not particularly limited as long as the grasping portion can grasp the fixation object. For example, the grasping portion may be formed into a clip shape.

Moreover, regarding the measuring device 80, the fixing member 81 includes the grasping portion for grasping the finger or the wrist, and the single electrode 82 is mounted thereon. Accordingly, it is possible to flexibly deal with the variation in the number of places of measurement, by merely fixing as many of the measuring devices 80 as the number of the places of measurement. In addition, the measuring devices 80 can easily change the distance between the electrodes 82 for measurement by merely changing the positions on the finger or the wrist where the fixing members 81 are fixed.

### (Modified Examples)

In Fig. 1 to Fig. 11, the voltage measured by the electrode 12, 22a, 22b, or 82, the voltage amplified by the amplifier 34, 54, 64, or 74, the result of speech recognition acquired through the speech recognition processing performed by the computing unit 45, and the like are transmitted externally by the lead wire 13, 23, 33, 43, 53, 63, 73, or 83. However, instead of the lead wire, the measuring device 10, 20 or 80, or the active electrode-type measuring device 30, 40, 50, 60, or 70, for example, a wire such as a shield cable, or a communicating unit of a wireless type may be mounted.

In addition, the measuring device 10, 20, or 80, or the active electrode-type measuring device 30, 40, 50, 60, or 70 may be designed to exchange signals with the external device not only by transmitting to the external device the voltage measured by the electrode, the voltage amplified by the amplifier, and the result of speech recognition by the computing unit, but also by receiving an instruction signal from the external device and transmitting a response signal relevant thereto, by use of the wire such as the lead wire or the shield cable and the like, or the communicating unit of the wireless type.

Moreover, the fixing members 11, 21, 31a, 31b, 41a, 41b, 51, 61, 71, and 81 are made of the nonconductive material. However, only the contact surface of the fixing member with the fixation object needs to be made of the nonconductive material. Therefore, parts other than the contact surface of any of the fixing members may be made of conductive material. Furthermore, the shapes of the fixing members 11, 21, 31a, 31b, 41a, 41b, 51, 61, 71, and 81, the shapes of the electrodes 12, 22a, 22b, 32a, 32b, 42a, 42b, 52, 62, 72, and 82, and the shapes of the amplifiers 34, 44, 54, 64, and 74 are not limited to the shapes shown in Fig. 1 to Fig. 11. The fixing member needs only to be capable of fitting to the fixation object or grasping the fixation object. The electrode needs only to be capable of being pressed to the measurement surface against the measurement object.

Moreover, it is also possible to mount a layer made of the nonconductive material on the measurement surface side of any of the electrodes 12, 22a, 22b, 32a, 32b, 42a, 42b, 52, 62, 72, and 82 which contacts the measurement object. Moreover, regarding the measuring devices 10, 20, and 80, which are shown in Fig. 1, Fig. 4, and Fig. 11 respectively, it is also possible to incorporate a preamplifier into any of the electrodes 12, 22a, 22b, and 82 to prevent noises from mixing into the measured voltage.

It is to be noted that the scope of the present invention is not limited to the above-described embodiment, and is defined by the appended claims.

## Claims

1. A measuring device for biological information, comprising:
(a) an annular fixing member (21) configured to be attached to a finger of a human body and being made of a non-conductive material;
***characterized by***
(b) an amplifier configured to amplify measured voltages;
(c) a plurality of annular electrodes (22a, 22b) for voltage measurement provided in contact with the outer circumference of the annular fixing member (21), wherein an interval (21a) is provided between adjacent electrodes (22a, 22b), respectively and adjacent electrodes are attached to the amplifier; and
(d) a transmitter configured to transmit the measured voltage externally.

2. The measuring device according to claim 1, further comprising a computing unit configured to process the output signal of the amplifier.

## Patentansprüche

1. Ein Messgerät für biologische Information, umfassend:
(a) ein ringförmiges Befestigungsglied (21), das konfiguriert ist, an einem Finger eines menschlichen Körpers angebracht zu werden, und aus einem nicht-leitenden Material hergestellt ist;
**gekennzeichnet durch**
(b) einen Verstärker, der konfiguriert ist zum Verstärken von gemessenen Spannungen;
(c) eine Vielzahl von ringförmigen Elektroden (22a, 22b) für Spannungsmessung, bereitgestellt in Kontakt mit dem äußeren Umfang des ringförmigen Befestigungsglieds (21), wobei ein Intervall (21a) bereitgestellt wird zwischen benachbarten Elektroden (22a, 22b), und benachbarte Elektroden an dem Verstärker angebracht sind; und
(d) einen Sender, der konfiguriert ist zum Übertragen der gemessenen Spannung nach extern.

2. Das Messgerät nach Anspruch 1, ferner umfassend eine Berechnungseinheit, die konfiguriert ist zum Verarbeiten des Ausgangssignals des Verstärkers.

## Revendications

1. Dispositif de mesure pour information biologique, comprenant :
(a) un élément annulaire de fixation (21) configuré pour être attaché au doigt d'un corps humain et étant constitué d'un matériau non conducteur ;
**caractérisé par**
(b) un amplificateur configuré pour amplifier des tensions mesurées ;
(c) une série d'électrodes annulaires (22a, 22b) pour la mesure de tension, mises en contact avec la circonférence extérieure de l'élément annulaire de fixation (21), où un intervalle (21a) est prévu entre les électrodes adjacentes (22a, 22b), respectivement et les électrodes adjacentes sont attachées à l'amplificateur, et
(d) un transmetteur configuré pour transmettre la tension mesurée vers l'extérieur.

2. Dispositif de mesure selon la revendication 1, comprenant en outre une unité de calcul configurée pour traiter le signal de sortie de l'amplificateur.
